# EUROPEAN PATENT APPLICATION

(11) **EP 4 603 016 A2**
(43) Date of publication of application: **20.08.2025**
(21) Application number: 25188119.9
(22) Date of filing: 30.12.2022
(51) Int. Cl.: A61B 5/145

(54) **A WEARABLE DEVICE FOR CONTINUOUS MONITORING OF HEALTH PARAMETERS**

(30) Priority: 30.12.2021 EP 21383240; 30.12.2021 EP 21383241
(62) Divisional of application: 22844558.1
(71) Applicant: Onalabs Inno-Hub, 08290 Cerdanyola del Valles (ES)
(72) Inventor: RABOST GARCÍA, Genís, 08290 Cerdanyola del Valles (ES); MUÑOZ PASCUAL, Francesc Xavier, 08290 Cerdanyola del Valles (ES); AGUILAR TORÁN, Javier, 08290 Cerdanyola del Valles (ES); PUNTER VILLAGRASA, Jaime, 08290 Cerdanyola del Valles (ES); COLMENA RUBIAL, Valeria, 08290 Cerdanyola del Valles (ES)
(74) Representative: Hoffmann Eitle

(57) **Abstract**

The present invention refers to a device for the continuous monitoring of health condition of patients or sport persons without the need of blood extraction. In particular the invention refers to a wearable device that comprises: at least one sweat sensor for measuring a sweat biomarker, a sweat collection inlet arranged in the device for collecting sweat when the device is worn by a user, a microfluidic channel for conveying collected sweat from the inlet to the sweat sensor, at least one vital-sign sensor arranged in the device for measuring a vital-sign or physiological sign of the user, a sweat volume sensor for measuring volume of the collected sweat, and a processing unit adapted for receiving and processing data provided by the sweat sensor, the vital-sign biosensor and the sweat volume measuring device. The invention can advantageously be used in sports medicine and/or sports health sectors for remote effort and/or fatigue assessment.

## Description

This is a divisional application from patent application EP 22 844 558.1.

### Field and object of the invention

The present invention belongs to the technical filed of medical devices, in particular to the area of wearable medical devices both for clinical and sport applications.

An object of the invention, is the provision of a multi-sensor device for the continuous remote, and real-time monitoring of health condition of patients or sport persons, without blood extraction and a method thereof.

The invention can advantageously be used in sports medicine and/or sports health sectors for remote effort and/or fatigue assessment.

### Background of the invention

Blood tests are the reference clinical methods that show the most important physiological parameters that allow an accurate diagnosis of a patient's condition. In the health sector, the analysis of most of the metabolites of interest (various amino acids, sugars, carboxylic acids and fatty acids, ...) requires blood collection, creating an obstacle for their use in real-time monitoring in both clinical health and sports medicine.

Therefore, there is a need in the medical personnel in charge of clinical protocols as well as in the training and recovery of the athletes, for devices capable of monitoring biomarkers in a non-invasive, continuous and remote way by means of sweat. In this regard, although the use of blood is the reference, analysing sweat has been a challenge to obtain bioequivalence between both measurement formats.

The current clinical trend is to be less invasive contemplates the tendency to combine both non-invasive measurement techniques: on skin and on biofluid (urine, saliva, sweat, etc.).

To measure sweating volume and rate, there are different strategies like: using humidity sensors, calorimetric measurements or volumetric determination. Using humidity sensors or calorimetric sensors are highly sensitive, but require a high level of complexity that limits their integration into portable devices. In contrast, volumetric measurements, determining the volume collected in a controlled period of time, allows simple and direct monitoring. There are visual monitoring options that are simple for the research setting as they require little instrumentation. However, for continuous monitoring applications it is better to use impedimetric detection (electrical resistance is reduced proportionally to the sample volume) which allows autonomous and real-time measurement.

Also, there are many types of wearable devices that can be worn by a user to continuously monitor an individual's activities, such as walking or running without unduly interrupting or limiting those activities. These wearable devices include electronics, and physiological sensors configured to sense certain physiological parameters of the wearer, such as heart rate, as well as motion sensors, GPS, etc.

These known devices generally have the same format, and are adapted to monitor only one physiological parameter of the wearer, and have limited capacities in terms of electronic processing, and communications capacities.

There is therefore an unmet medical need for non-invasive devices and methods of determining the concentration of biomarkers in blood in a cheap fast and accurate way.

### Summary of the invention

The present invention is defined in the attached independent claim, and satisfactorily solves the drawbacks of the prior art, by providing a wearable device that integrates different sensors to obtain simultaneous measurements of biomarkers in sweat (metabolites, ions or amino acids, etc.), and vital physiological parameters (heart and respiratory rate, blood pressure, etc.), for the determination of lactate concentration in blood, in a non-invasive, continuous, real-time and remote manner.

More specifically, a first aspect of the invention refers to a wearable device for continuous monitoring of health parameters of a user, wherein the device comprises: at least one sweat sensor for measuring a sweat biomarker, a sweat collection inlet arranged in the device for collecting sweat when the device is worn by a user, and a microfluidic channel for conveying collected sweat from the inlet to the sweat sensor.

Additionally, the device comprises: at least one vital-sign sensor arranged in the device for measuring a vital-sign or physiological sign of the user, a sweat volume sensor for measuring volume of the collected sweat, and a processing unit adapted for receiving and processing data provided by the sweat sensor, the vital-sign biosensor and the sweat volume measuring device.

Preferably, the vital-signs or signs sensors is one or more of the following sensors: a heart rate sensor, a respiratory rate sensor, a blood pressure sensor, a body temperature sensor, and an oxygen saturation sensor.

The processing unit is further adapted to calculate or predict a blood lactate concentration based on data provided by the sweat sensor, the sweat volume sensor, and a vital sign-sensor. Preferably, the data is the form of electric signals that can be processed by the processing unit to perform calculations based on that data.

In a preferred embodiment, the sweat sensor is a sweat lactate sensor and the vital-sign sensor is a heart rate sensor, and the processing unit is further adapted to calculate, preferably by means of machine learning algorithms in a known manner for a skilled person in the art, predictive values of lactate concentration in blood, based on data in the form of electric signals, provided by: the sweat lactate sensor, the sweat volume sensor and heart rate sensor.

Therefore, the wearable device of the invention is a smart wearable device for lactate monitoring, that integrates a microfluidic system that ensures efficient sweat capture and continuous circulation of a fresh sweat sample. The invention is capable of providing real time health status of athletes and report when an athlete enters the anaerobic phase, thus, creating a new paradigm in sports training planning, as well as the health status of the athlete.

A direct and proportional relationship between lactate measurement (key biomarker in the monitoring of intensive physical exertion or fatigue) in blood and sweat is unknown, thus, in the present invention it has been found that taking into account additional parameters to sweat lactate, would establish a more accurate bioequivalence with blood lactate.

In particular, the invention involves the processing of the following additional parameters to sweat lactate for the blood lactate estimation: sweat rate, sweat volume excreted by the subject, and heart rate. In the invention, the sweating rate is also taken into account as a factor of dilution of lactate and the other analytes present in sweat.

The sweating rate gives an indication of the decrease in sweat lactate concentration in the tests and the variation observed with the blood lactate trend. Moreover, the heart rate provides a good estimation of the intensity of the physical activity performed by a user, and it is a common parameter in the monitoring of the athlete's condition and fatigue.

The wearable device of the invention integrates a microfluidic system for collecting sweat, that in contrast to prior art devices that are only capable of providing time isolated sweat measurements of no practical use, includes a sweat sample renewal system, such that fresh sweat measurement is always performed, which provide a faithful indication of the physical effort made at that time by a user.

Furthermore, the device incorporates a communication module adapted for the wireless transmission of data processed by the processing unit, preferably for transmitting the estimated or calculated blood lactate concentration.

The device is provided with a sensing chamber and at least one of the following sensors placed in the sensing chamber: a sweat lactate sensor, a sweat conductivity sensor, metabolites sensor, ions sensors, amino acids sensor. The microfluidic channel communicates the sweat inlet with the sensing chamber.

The sweat volume sensor comprises a microfluidic circuit or a microfluidic reservoir, fluidly communicated with the sensing chamber and arranged downstream the sensing chamber, and a pair of electrodes such that the microfluidic reservoir is arranged in between the two electrodes, in a way that a capacitance value between the two electrodes is variable depending on the amount of sweat in the reservoir. The two electrodes are a pair of strips opposite each other, and more preferably the electrodes are embodied as conductive flexible strips.

The microfluidic reservoir can have any form, for example the microfluidic reservoir can be a straight or curved conduit, or it can be formed as a conduit having a meander configuration.

Preferably, the wearable device comprises three main parts, namely: a main housing, means for attaching the main housing to a part of the user's body, and a fungible or consumable part disposable after use, which is configured to be manually attachable and detachable from the main housing.

The means for attaching the main housing to a part of the user's body, preferably consist of a flexible band having two ends respectively couplable with the main housing.

In a preferred embodiment, the processing unit is integrally contained in the main housing, but in other preferred embodiments of the invention, the processing unit or part of it, is external to the device, for example, it is implemented in a smart phone, a smart watch, a tablet or similar devices. The main housing additionally includes a power source like a battery, for supplying power to the electronics implementing the processing unit, and electric connectors for the communication with the consumable part.

The flexible band is fitted with at least one biosensor for measuring a vital-sign or physiological sign of the user, such as a heart rate sensor, thus, in addition to the sweat measurements, heart rate measurements are also taken in direct contact with the skin, with the same de device, and simultaneously as the sweat parameters are measured.

The flexible band is implemented as an elastic textile tape that would have a good hold on the body and would contain a pair of electrodes to measure the heart rate. The electrodes are made of bioelectric silicone to capture the electrocardiogram signal and extract the heart rate. They have sufficient conductivity to obtain a quality heart rate signal and are resistant to continuous use and washing.

The flexible band can be provided with other sensors, like a respiratory rate sensor to obtain additional parameters.

The consumable component has a contact surface provided to be in contact with the user's skin when the main housing is attached to a user's body part, and the consumable component is coupled with the main body.

Alternatively, the means for attaching the main housing to a part of a user's body, comprises an adhesive material for example an adhesive surface suitable to be adhered on a user's skin. For example, this adhesive surface is provided on a surface of the consumable component meant to be in contact with the user's skin.

The consumable component incorporates: the sweat collection inlet formed in the consumable component's contact surface at least one sweat sensor, the sweat rate measuring device, and the microfluidic channel, and electric connection means for electrically connecting the consumable component with the processing unit when the consumable component is coupled with the main housing.

Preferably, the consumable component is a generally flat body embodied as a card-like component.

In a preferred implementation of the wearable device, the main housing has a front surface and a back surface provided with a pair of guides opposite each other, and the consumable component has a pair of sides wings, such that the consumable component is couplable with the main housing by inserting its side wings respectively in the guides and by moving the consumable component on the back surface of the main housing.

A part of the back surface of the main housing is generally flat, and the consumable component is couplable with the main housing by moving the consumable component on a plane parallel to said generally flat part of the back surface.

The main body is provided by a pair of electric connectors and each end of the flexible band is fitted with metallic connectors for mechanically and electrically connecting the flexible band and the biosensor with the main body.

The main body includes a battery for supplying power to the processing unit, and the main body has a lid at its back surface for providing access to the battery. The consumable component is overlapped with the lid when the consumable component is operatively coupled with the main housing.

The wearable device of the invention, provides an advanced and integral personalized health management capability, through the combined analysis of key metabolic and physiological health indicators, like blood glucose measurement data, in addition to heart rate, oxygen saturation and temperature measurements, to achieve accurate equivalence with blood glucose measurements.

In summary, some of the main advantages of the invention are the followings:
- the diagnostic capacity of a biofluid like sweat is increased; much more patient data is generated by monitoring in a non-invasive, continuous and remote way;
- the format of the wearable device, is already used by athletes (heart rate monitor) to which they are accustomed in their regular training;
- the permanent part of the device (main housing) can be used by itself, in case the chemical measurement with the fungible or consumable part is not necessary (already known training routine, etc...);
- the permanent part (main housing) is compatible with different consumable part depending on the parameters to be measured. Useful tool not only for the end user but also for the clinic/sports physician who can adapt the device to the requirements of the patient/study;
- simple to use by insertion of the consumable and placement of the heart rate monitor. Guarantees placement in the same place every time, limiting the variability that the user's own operation may entail;
- it allows to have more advanced electronics with advanced functionalities compared to patch wearables such as battery management, data processing, flash memory in case there is no possibility of communicating the data;
- the consumable part might have an adhesive surface to enhance its fastening with the permanent part.

The main application of the invention is focused on sports medicine or sports health sector, for example for monitoring dehydration monitoring in athletes, but depending on the combination of sensors used, the device can be adapted to different applications as explained below.

Therefore, in a preferred embodiment, the wearable device is adapted to operate as a device for dehydration monitoring in athletes. A combination of sensors for measuring sweating, conductivity and some salt such as sodium can give real-time information on the state of dehydration of an athlete during physical exertion. Important to improve the control of dehydration in tests, and to be able to be treated in time (as an alarm system for the user) and with measurable variables in order to create an objective scale.

In another preferred embodiment, the wearable device comprises a heart rate and sweat rate measurement sensors, and the device is adapted to operate as a device for monitoring user fatigue.

In another preferred embodiment, the wearable device is adapted to operate as a device for nocturnal hypoglycaemia monitoring. In this embodiment, the device is a non-invasive device provided with sensors for measuring: heart rate, sweating, conductivity and a glucose sensor adapted to low ranges. The user would put the device on during the night, and the device can generate alarms when an episode of this type is detected. With the addition of predictive models, the onset of an episode could be predicted in advance to prevent and limit its consequences.

In another preferred embodiment, the wearable device is adapted to operate as a device for peritoneal dialysis remote monitoring. In this embodiment, the device comprises a heart rate sensor, an accelerometer, a sweat rate sensor, and sensors for measuring: conductivity, ions such as sodium, and lactate.

The present invention also relates to a wearable device for continuous monitoring of health parameters of a user. The device comprises at least one sweat sensor for measuring a sweat biomarker, a sweat collection inlet arranged in the device for collecting sweat when the device is worn by a user, a microfluidic channel for conveying collected sweat from the inlet to the sweat sensor, at least one vital-sign sensor arranged in the device for measuring a vital-sign or physiological sign of the user, a sweat volume sensor for measuring volume of the collected sweat, and a processing unit adapted for receiving and processing data provided by the sweat sensor, the vital-sign biosensor and the sweat volume sensor. The vital-sign sensor comprises at least a heart rate sensor; and the processing unit is further adapted to calculate, preferably by means of machine learning algorithms, the concentration of the biomarker in blood based on data provided by: the sweat biomarker sensor, the sweat volume sensor and heart rate sensor.

In a preferred embodiment, the sweat biomarker sensor is selected from a list comprising: a sweat lactate sensor and a sweat glucose sensor. In another preferred embodiment, the sweat biomarker sensor is a sweat lactate sensor.

More preferably, the sweat biomarker sensor is a sweat lactate sensor, and the processing unit is adapted to calculate, preferably by means of machine learning algorithms, a blood lactate concentration based on data provided by: the sweat lactate sensor, the sweat volume sensor and heart rate sensor.

In another preferred embodiment, the wearable device further comprises a sweat rate sensor, and the processing unit is further adapted for monitoring user fatigue.

In another preferred embodiment, the wearable device further comprises a conductivity sensor and a ion sensor, and the processing unit is further adapted for monitoring dehydration in athletes, based on data provided by the sweat volume sensor, the conductivity sensor and the ion sensor.

In another preferred embodiment, the sweat biomarker sensor is a sweat glucose sensor and further comprising: a conductivity sensor, and the processing unit is adapted to calculate, preferably by means of machine learning algorithms, a blood glucose concentration based on data provided by the heart rate sensor, the sweat volume sensor, the conductivity sensor and the sweat glucose sensor. More preferably, the processing unit is further adapted to monitor the nocturnal hypoglycaemia, based on data provided by the heart rate sensor, the sweat volume sensor, the conductivity sensor and the sweat glucose sensor. Even more preferably, the sweat glucose sensor is adapted to detect glucose sweat concentrations below 55mg/L.

In another preferred embodiment, sweat biomarker sensor is a sweat lactate sensor and/or a sweat glucose sensor, the device further comprising an accelerometer, a conductivity sensor, and an ions sensor preferably a sodium sensor, and wherein the processing unit is further adapted to monitor the peritoneal dialysis based on data provided by the heart rate sensor, the accelerometer, the sweat rate sensor, the conductivity sensor, the ions sensor and the sweat lactate sensor and/or the sweat glucose sensor.

A second aspect of the invention refers to a method for non-invasively determining the concentration of a biomarker in blood, the method comprising:
a) receiving the concentration of such biomarker in sweat, the volume of sweat received and the heart rate of the subject,
b) computing through a multiparametric regression model a value representing the concentration of the biomarker in blood, and
c) determining the computed value is the concentration of the biomarker in blood.

In a preferred embodiment, the biomarker is selected from a list comprising: lactate and glucose. In another preferred embodiment, the biomarker is lactate.

In another preferred embodiment, the biomarker is lactate and the method comprises determining the blood lactate concentration based on the sweat lactate, the sweat volume and the heart rate.

In another preferred embodiment, the concentration of the biomarker in sweat, the volume of sweat received, and/or the heart rate of the subject are received from a wearable device according to any of the embodiments of the first aspect of the invention.

A third aspect of the invention refers to a method for non-invasively or minimally invasively evaluating the condition of a subject, the method comprising determining the concentration of a biomarker in blood in a non-invasive or minimally invasive way, based on the concentration of such biomarker in sweat.

In a preferred embodiment, the method comprises determining the concentration of the biomarker in blood based on the concentration of such biomarker in sweat, the sweat volume, and the heart rate of the subject.

In another preferred embodiment, the biomarker is selected from a list comprising lactate and glucose. In another preferred embodiment, the biomarker is lactate.

In another preferred embodiment, the concentration of the biomarker in sweat, the volume of sweat received, and/or the heart rate of the subject are received from a wearable device according to any of the embodiments of the first aspect of the invention.

### Brief description of the drawings

Preferred embodiments of the invention are henceforth described with reference to the accompanying drawings, wherein:
Figure 1.- shows a perspective view of a preferred embodiment of the invention including an attachment band or strap.
Figure 2.- shows a perspective view of the main housing and the consumable part partially coupled together.

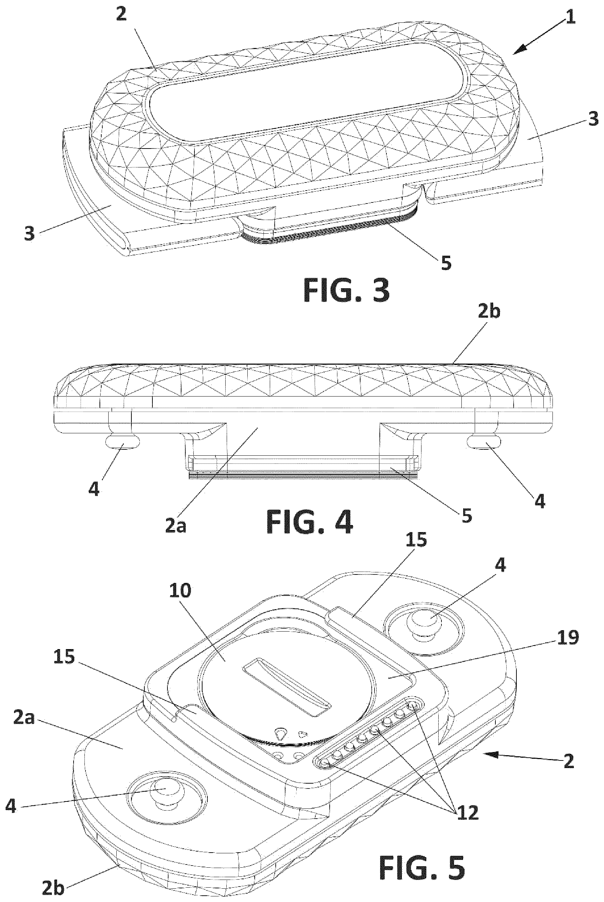

Figure 3.- shows another perspective view of the main housing from above, and part of the band ends.
Figure 4.- shows a front elevational view of the main housing.
Figure 5.- shows a perspective view of the main housing from below.
Figure 6.- shows an exploded view of the main housing components.
Figure 7.- shows in Figure A a perspective view of the main housing from below. In Figure B with the consumable part partially coupled, and in Figure C with the consumable part fully coupled.
Figure 8.- shows an schematic representation of the sweat volume sensor. The figure represents four stages (A - B) of filling of the microfluidic channel.
Figure 9.- shows a perspective view of the consumable device from the face opposite the surface meant to be in contact with the skin.
Figure 10.- shows a schematic representation in plan view of the consumable device.
Figure 11.- shows a cross-sectional view of the consumable device, taken at cutting plane A-A in figure 10.
Figure 12.- shows another cross-sectional view of the consumable device, taken at cutting plane B-B in figure 10.
Figure 13.- shows a graph illustrating the characterization of the sweat volume sensor.
Figure 14.- shows a graph illustrating the effects of sweat conductivity on measured capacitance of the sweat volume sensor.
Figure 15.- shows in Figure A a scheme of the physical tests carried on in the Example and in Figure B a diagram of the machine learning data distribution for prediction according to one or more embodiments of the invention.
Figure 16.- shows in Figure A the characterization graph of a lactic acid concentration sensor and in Figure B a graph comparing the current measurement for the reference potentiostat and the custom (proposed) potentiostat, according to one or more embodiments of the invention.
Figure 17.- shows in Figure A, an in vivo example of the working mechanism of the sweat volume sensor and in Figure B a graph illustrating the in-vitro characterization of a sweat rate sensor according to one or more embodiments of the invention.
Figure 18.- shows in Figure A a graph illustrating the Correlation plot between two blood lactate measurement systems, and in Figure B a Bland-Altman plot illustrating the agreement between both systems.
Figure 19.- shows a graph illustrating the relation of blood lactate versus sweat lactate values.
Figure 20.- shows a table depicting the root-mean-square-error (RMSE) for the different multiparametric approaches tested.
Figure 21.- shows a plot illustrating the correlation plot of the Multi-Layer Perception (MLP) model.
Figure 22.- shows in Figure A a plot illustrating the correlation plot of the Multi-Layer Perception (MLP) model of Figure 21 when the Lactate concentration is truncated at 10.5 Mm, and in Figure B two examples of continuous monitoring of blood lactate based on sweat according to one or more embodiments of the invention.
Figure 23.- shows a plot illustrating for the MLP model the importance inside the model of each input parameter.

### Preferred embodiment of the invention

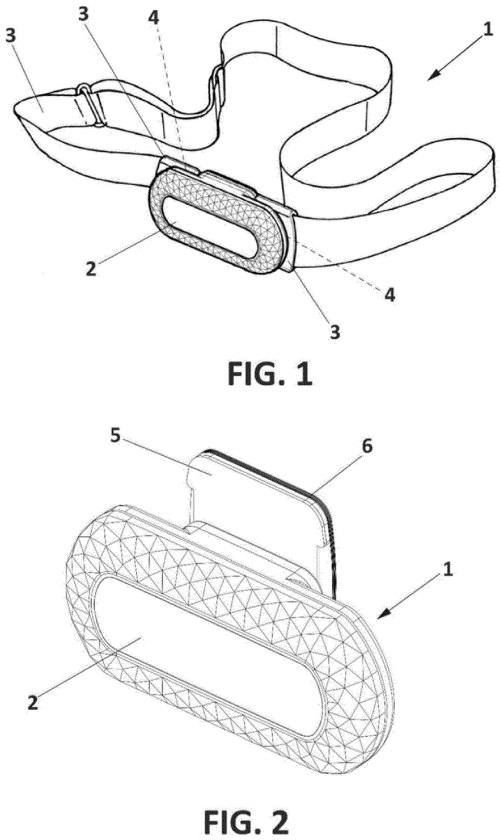

**Figure 1** shows an exemplary embodiment of a wearable device (1) according to the invention comprising a main housing (2) and a flexible band or strap (3) for attaching the main housing to a part of a user's body, and a pair of electric and mechanic connectors or snap buttons (4) provided in the main housing (2) for mechanically and electrically connecting the flexible band ends with the main housing (2).

The flexible band (3) is implemented as an elastic textile tape with female connectors at its ends to be coupled with the snap buttons (4). The flexible band (3) is fitted conventionally with at least one biosensor for measuring a vital-sign or physiological sign of the user, in a known manner for example a pair of electrodes to measure heart rate. The electrodes are made of bioelectric silicone to capture the electrocardiogram signal and extract the heart rate, and they have sufficient conductivity to obtain a quality heart rate signal and are resistant to continuous use and washing. The vital-signs or signs sensors are at least one of the following: a heart rate sensor, a respiratory rate sensor, blood pressure sensor, body temperature sensor, and oxygen saturation sensor.

As shown more clearly in **Figure 2****,** the device (1) includes a consumable component (5) configured to be manually coupled and uncoupled with the main housing (2), and having a contact surface (6) provided to be in contact with the user's skin when the consumable component (5) is coupled with the main housing (2), and the main housing (2) is attached to a user's body for example by means of the flexible band (3).

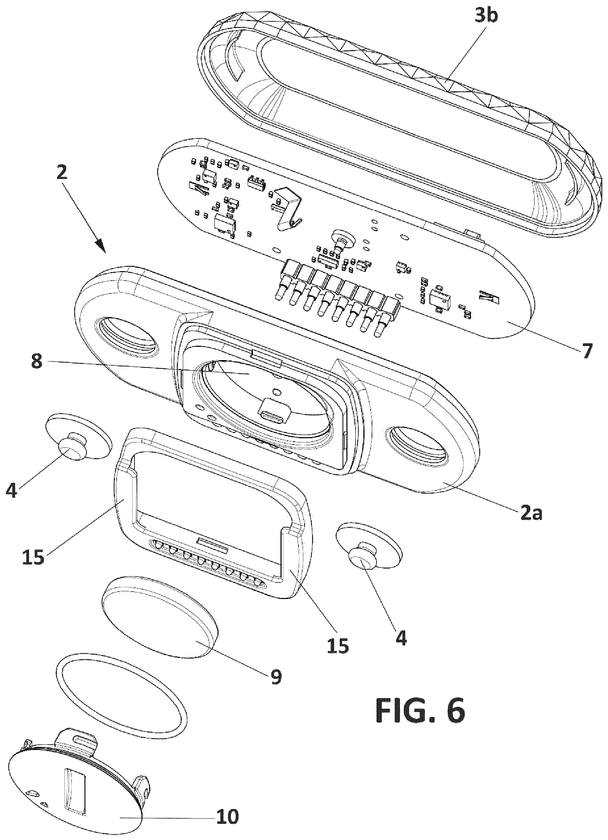

As shown in **Figure 6****,** the main housing (2) is formed by two couplable parts, a base (2a) and a lid or cover (2b) that configure a space within which an electronic circuit (7) is enclosed. This electronic circuit (7) implements the processing unit, sensors instrumentation, battery management, data processing and communication module for the wireless transmission of data processed by the processing unit.

The main housing (2) in particular the base (2a) has a receptacle (8) for receiving a battery (9) for supplying power to the electronic circuit (7), and a lid (10) for closing and opening the receptable (8).

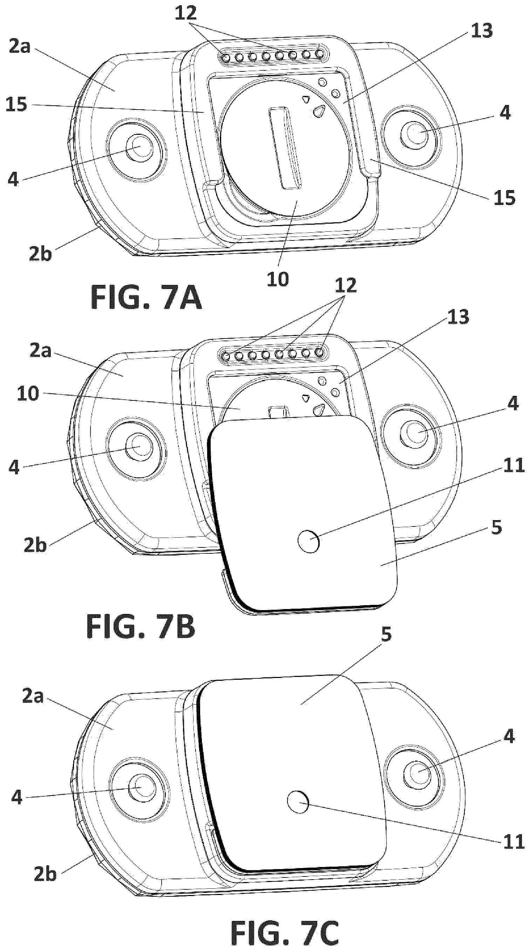

The consumable component (5) is generally a flat body, and the main housing (2) and the consumable component (5) are configured, such that the consumable component (5) is couplable with the main housing (2) by moving the consumable component (5) on the same lane as the generally flat back surface (19) of the main housing (2) or a plane parallel to it, as illustrated in **Figures 2****,** **7B** and **7C****,** such that the lid (10) can only be accessed when the consumable component (5) has been taken off from the main housing (2).

In particular, the consumable component (5) is overlapped with the lid (10) when the consumable component (5) is operatively coupled with the main housing (2), as it can be observed in **Figures 7A - 7C****.**

The consumable component (5) incorporates: a sweat collection inlet (11) formed in the consumable component's contact surface (6) for collecting sweat when the device is worn by a user, at least one sweat sensor, a sweat volume measuring device, and a microfluidic channel for conveying collected sweat from the inlet (11) to the sweat sensor.

In particular, consumable component (5) has a sensing chamber and at least one of the following sensors: a sweat lactate sensor, a sweat conductivity sensor, metabolites sensor, ions sensors, amino acids sensor, and, placed in the sensing chamber. The microfluidic channel communicates the sweat inlet (11) with the sensing chamber.

The consumable component (5) is manufactured as a stack of layers of plastic materials where the microfluidic channels are formed by laser cutting or die cutting. The integrated sensors are electrochemical in nature and therefore require electrodes that are fabricated by screen printing.

A set of electric connectors (12) are provided at the back surface (13) of the base (2a) of the main housing (2), in order to realise electric communication between the sensors located in the consumable component (5) and the processing unit (7). These connectors (12) are known spring-biased connectors, that stablish electric contact with corresponding electric pads (17) (provided in the consumable component (5), in a known manner when this is coupled with the main housing (2).

The consumable component (5) can be mechanically coupled and uncoupled from the main housing (2), in a quick and intuitive manner for a user, while ensuring functionality and good electrical connection with the main housing.

The main housing (2) has a back surface (13) provided with a pair of guides (15) opposite each other, in a way that a space or pocket is formed in between the guides (15) for receiving the consumable component (5) therein. In turn the consumable component (5) has a pair of sides wings (16), dimensioned and configured to fit in the space formed in between the guides (15), such that the consumable component (5) is couplable with the main housing (2) by inserting its side wings (16) respectively in the guides (15), and by moving the consumable component on the back surface (13) of the main housing (2) as represented in the sequence of **Figures 7A, 7B,** and **7C****..**

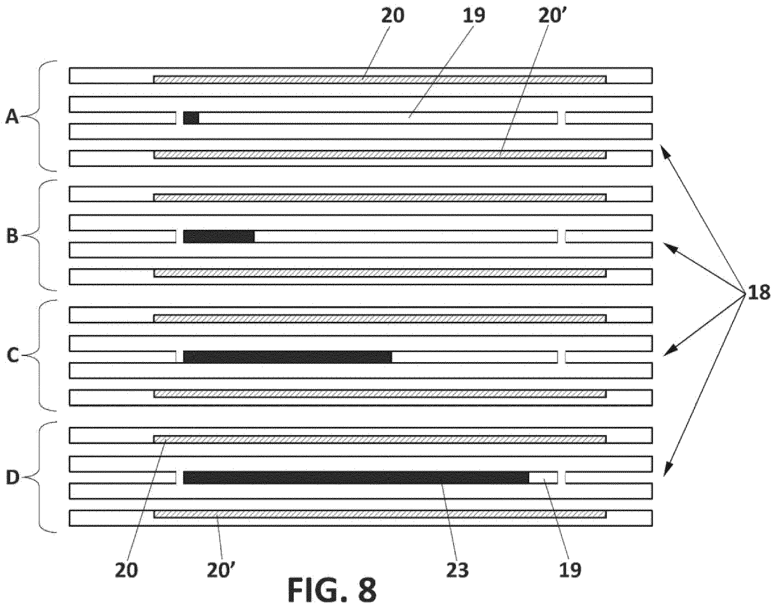

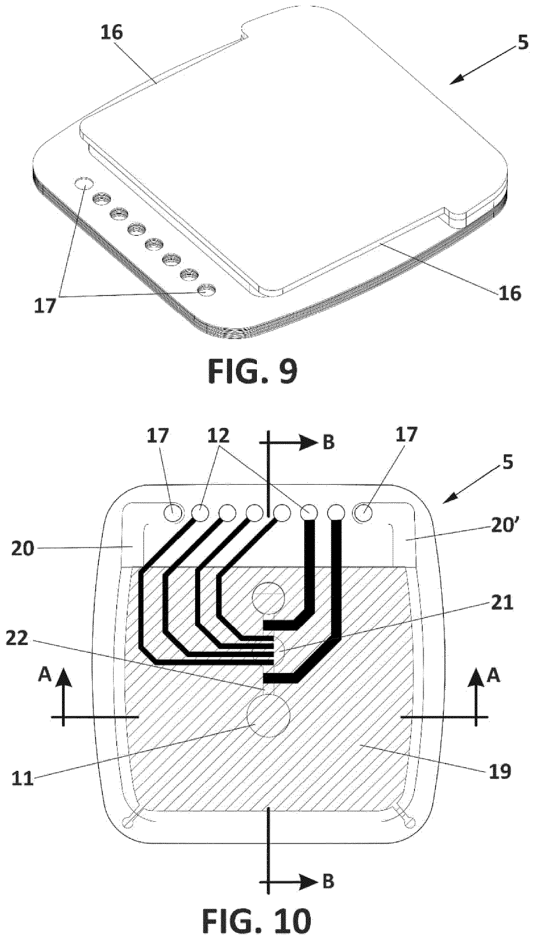

The sweat volume sensor (18) is represented schematically in **Figure 8****,** and comprises a microfluidic circuit or a microfluidic reservoir (19), fluidly communicated with the sensing chamber and arranged downstream the sensing chamber (21), such that sweat would enter the inlet (11), and flow along the microfluidic channel (22) and the sensing chamber (21) to gradually fill the reservoir (19), as more clearly represented in **Figure 10****.** In addition, the sweat volume sensor (18) comprises a pair of electrodes (20,20') and the microfluidic reservoir (19) arranged in between the two electrodes, such that a capacitance value between the two electrodes is variable depending on the amount of sweat in the reservoir.

The entry of sweat into the microfluidic reservoir (19) results in a change in capacitance between the two electrodes (dielectric constant difference between air and water which is the main component of sweat). This approach, apart from giving a very robust response with the added volume, has integration advantages, as it is only necessary to add two layers of copper tape in the lamination of the device, which is a cheap and easily available material. In addition, it does not require the fabrication of electrodes inside the channel and has sensitivity over the entire measurement area of the copper layers.

The electrodes (20,20') allow determination of the flow of sweat as it advances filling the reservoir (19) with limited influence of the ionic strength of the sweat sample.. The local sweat flow for each measurement interval can be estimated (we know the time and volume of sweat advancement) and thus be able to correct the sweat lactate measurement as a dilution factor.

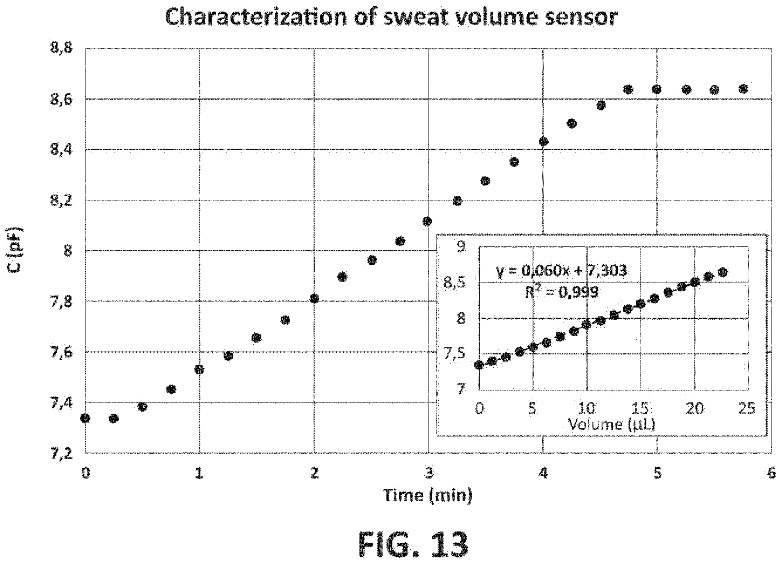

The experimental validation of this novel sweat volume sensor has been carried out by injecting a controlled volume with a syringe pump into the microfluidic reservoir while monitoring the capacitance between the two electrodes. The microfluidic reservoir consisted in a long serpentine microfluidic channel, fabricated using adhesive materials and laser cutting, entrapped between the electrodes made of copper conductive tape. A 100 mM NaCl solution, which replicates the typical ionic strength of sweat, was injected at a constant flow rate of 5 µL/min. In **Figure 13****,** it can be seen that, as the injection starts, the capacitance starts increasing linearly until the solution reaches the end of the sensing region. Then, the capacitance will remain stable although we keep injecting fluid because the sensing region is already completely filled. Converting time to volume injected, the calibration curve between capacitance and volume for the sweat volume sensor can be build.

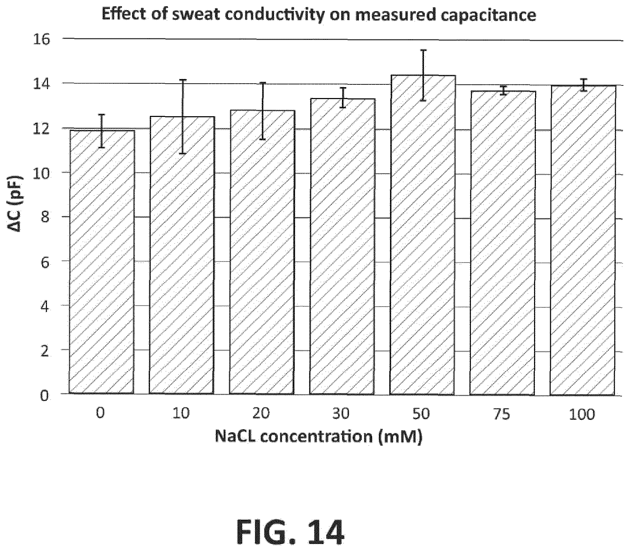

Most impedimetric sweat volume or rate sensors have a response which depends significantly on the conductivity or ionic strength of the sweat sample. This is because they have a resistive nature, where free ions present in sweat have a greater effect than in capacitive measurements, as the sensor proposed here. This can be observed in **Figure 14** where the increment in capacitance (between an empty and filled sweat volume sensor) is measured for different solutions of increasing ionic strength (NaCl solutions ranging from 0 to 100 mM). There is a slight increase in the increment of capacitance when the solution contains a higher concentration of ions, but for the range of interest (low, 30 mM, to high, 100 mM, concentrations) the change produced by the conductivity of the sample is found between the margins of the variability of the sensor.

Preferably, the two electrodes are a pair of conductive flexible strips opposite each other.

The consumable component (5) can be manufactured as a stack of layers of plastic materials where the microfluidic channels and sensing chamber are formed by laser cutting or die cutting. The integrated sensors are electrochemical in nature, and therefore require electrodes that are fabricated by screen printing.

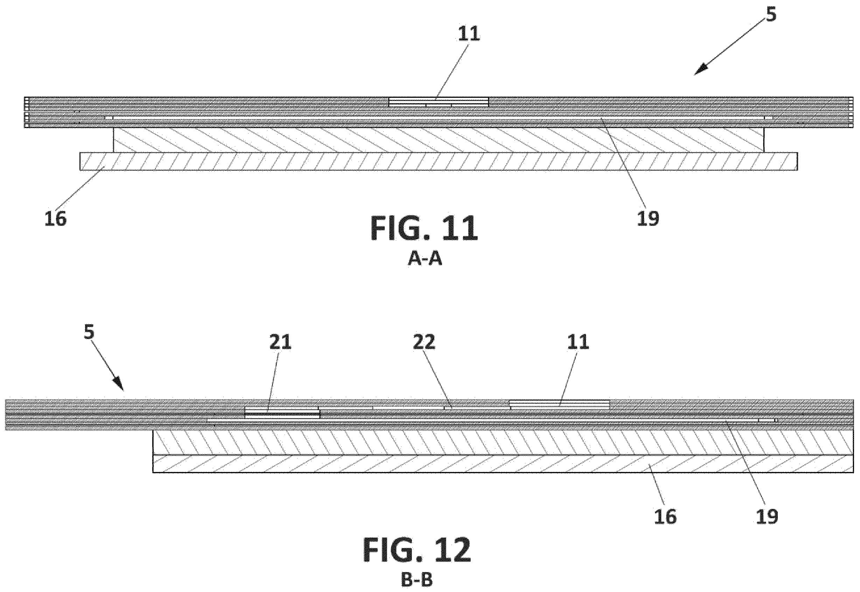

As shown in **Figures 11** and **12****,** in this particular embodiment, the sweat inlet (11), the microfluidic channel (22) and the sensing chamber (21), are placed above the microfluidic reservoir (19).

Also comprised within this invention, it is a wearable device (1) for continuous monitoring of health parameters of a user,. The device (1) comprises at least one sweat sensor for measuring a sweat biomarker, a sweat collection inlet (11) arranged in the device for collecting sweat when the device is worn by a user, a microfluidic channel (22) for conveying collected sweat from the inlet to the sweat sensor, at least one vital-sign sensor arranged in the device for measuring a vital-sign or physiological sign of the user, a sweat volume sensor (18) for measuring volume of the collected sweat, and a processing unit (7) adapted for receiving and processing data provided by the sweat sensor, the vital-sign biosensor and the sweat volume sensor (18).

According to this embodiment the vital-sign sensor comprises at least a heart rate sensor; and the processing unit (7) is further adapted to calculate, preferably by means of machine learning algorithms, the concentration of the biomarker in blood based on data provided by: the sweat biomarker sensor, the sweat volume sensor (18) and heart rate sensor.

More preferably, the sweat biomarker sensor is selected from a list comprising: a sweat lactate sensor and a sweat glucose sensor. Thus, the device (1) is configured for monitoring the concentration of lactate and/or glucose in blood based on data provided by the respective sweat biomarker sensor, the sweat volume sensor and heart rate sensor.

More preferably, the sweat biomarker sensor is a sweat lactate sensor, and the processing unit is adapted to calculate, preferably by means of machine learning algorithms, a blood lactate concentration based on data provided by: the sweat lactate sensor, the sweat volume sensor and heart rate sensor.

A second aspect of the invention refers to a method for non-invasively determining the concentration of a biomarker in blood , the method comprising:
a) receiving the concentration of such biomarker in sweat, the volume of sweat received and the heart rate of the subject,
b) computing through a multiparametric regression model a value representing the concentration of the biomarker in blood, and
c) determining the computed value is the concentration of the biomarker in blood.

In some embodiments wherein the computed value of step b) is not in a standardised scale, the method may comprise an intermediate step before determining the concentration of the biomarker in blood, comprising computing a standardised value representing the concentration of the biomarker in blood from the computed value of step b).

In a preferred embodiment of the second aspect of the invention, the biomarker is selected from a list comprising or consisting of: lactate and glucose.

Therefore, the method is for non-invasively determining the concentration of lactate or glucose in blood, such that when the concentration of lactate or glucose in sweat, the volume of sweat received and the heart rate of the subject is received, a value representing the concentration of lactate or glucose in blood can be computed respectively , and it can be finally determined that the computed value is the concentration of lactate in blood or the concentration of glucose in blood, respectively.

In a preferred embodiment of the second aspect of the invention, the biomarker is lactate. Advantageously, this allows for the non-invasive monitoring of blood-related conditions associated to lactate. As shown in Example 1, the determined concentration of lactate in blood from the non-invasive measurement of lactate in sweat, the sweat volume and the heart rate of the subject has not only high correlation with the determined concentration of lactate in blood when measured directly, but the provided values are standardized values for the concentration of lactate in blood. Therefore, the method is for non-invasively determining the concentration of lactate o in blood, such that when the concentration of lactate in sweat, the volume of sweat received and the heart rate of the subject is received, a value representing the concentration of lactate in blood can be computed, and it can be finally determined that the computed value is the concentration of lactate in blood.

In another preferred embodiment of any of the embodiments of the second aspect of the invention, the concentration of such biomarker in sweat, the volume of sweat received and/or the heart rate of the subject is obtained from a device (1) according to any of the embodiments of the first aspect of the invention.

A third aspect of the invention refers to a method for evaluating the condition of a subject, the method comprising determining the concentration of a biomarker in blood in a non-invasive or minimally invasive way, based on the concentration of such biomarker in sweat.

More preferably, the method comprises determining the concentration of a biomarker in blood in a non-invasive or minimally invasive way, based on the concentration of such biomarker in blood, the sweat volume, and the heart rate of the subject. More preferably, the biomarker is lactate or glucose.

In a preferred embodiment of the third aspect of the invention, the biomarker is selected from a list comprising or consisting of: lactate and glucose.

In another preferred embodiment of any of the embodiments of the third aspect of the invention, the concentration of such biomarker in sweat, the volume of sweat received and/or the heart rate of the subject is obtained from a device (1) according to any of the embodiments of the first aspect of the invention.

The invention is described below by the following examples, which must be considered as merely illustrative and in no case limiting of the scope of the present invention.

### EXAMPLE: BLOOD LACTATE FROM NON-INVASIVE MEASUREMENTS

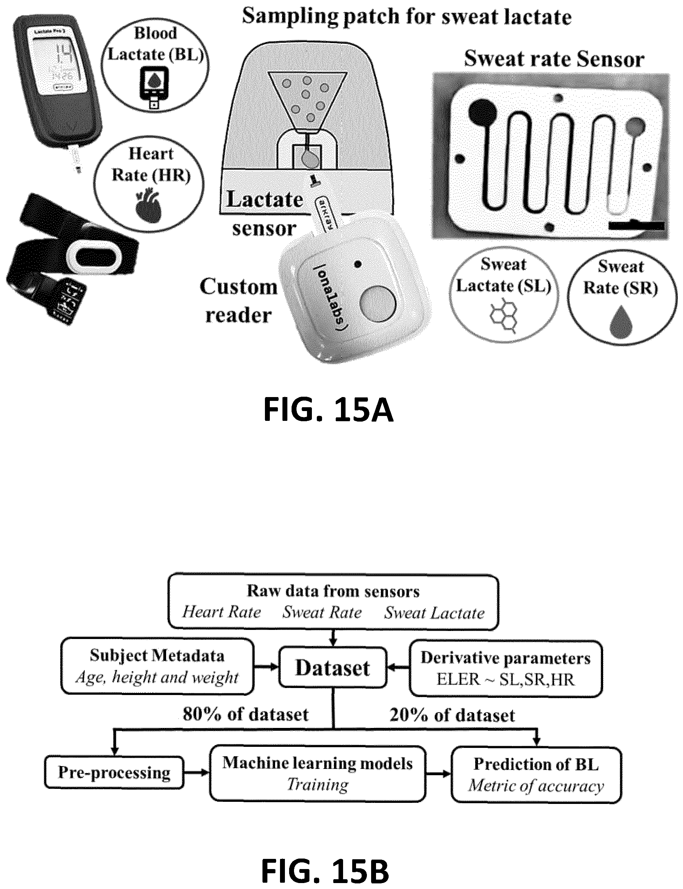

A bioequivalence study was carried out in order to test the feasibility of blood lactate prediction using solely non-invasive measurements. Sweat lactate was measured using a commercial lactate sensor adapted for the range of concentrations found in sweat, custom wireless instrumentation and a microfluidic sampling patch. Sweat rate was measured from a sweat volume sensor already validated in other works which rely on the visual inspection of the sweat front inside a microfluidic channel. Heart rate was measured using a heart rate monitor (chest strap). Blood lactate was measured using portable meters which use single-use test strips as well as the colorimetric reference instrumentation found in the lab. All these measurements were carried out simultaneously during a physical test with volunteers (Figure 15A).

These measurements, plus subject metadata (age, height and weight) and derivative parameters such as ELER (Exhaustion and Lactate Excretion Rate), produced a dataset that was used to predict blood lactate using machine learning algorithms (Figure 15B). ELER parameter consisted in a combination of Sweat Lactate (SL), Sweat Rate (SR) and Heart Rate (HR) to provide the model with relations between variables with physiological context. For example, SR is used to correct the factor of dilution of sweat lactate in a sample of irregular generation. HR helps providing the context of the user in terms of exercise intensity and fatigue.

Therefore, the goal in this study was to test the capability of a multiparametric model of predicting blood lactate with enough accuracy, overcoming the challenges associated to sweat analysis and lactate monitoring, in particular. In fact, recent wearable technology was used to replicate tools that could be applied in a real scenario combined with the rigorousness of physiological studies.

### In vitro characterization

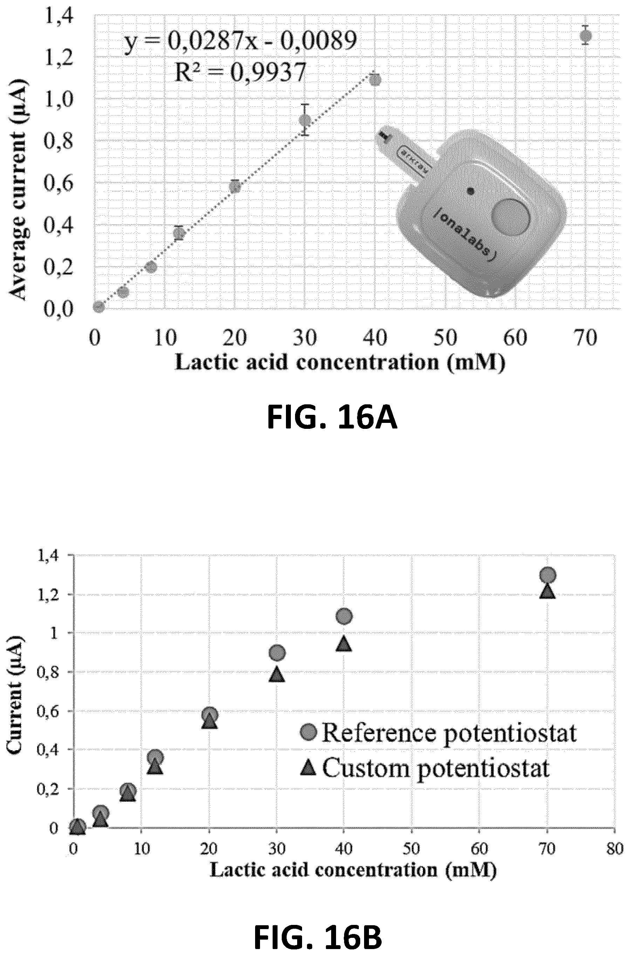

The sweat sensors used in this study were characterized initially at the laboratory. The sweat lactate sensor was characterized using artificial sweat samples, to replicate the sample matrix and interferents in the sensor response, in the concentration range of interest. We could observe that the sweat sensor used could measure up to 40 mM with a good linear response (Figure 16A). Besides, our custom instrumentation provided values comparable to commercial potentiostats (Palmsens) (Figure 16B).

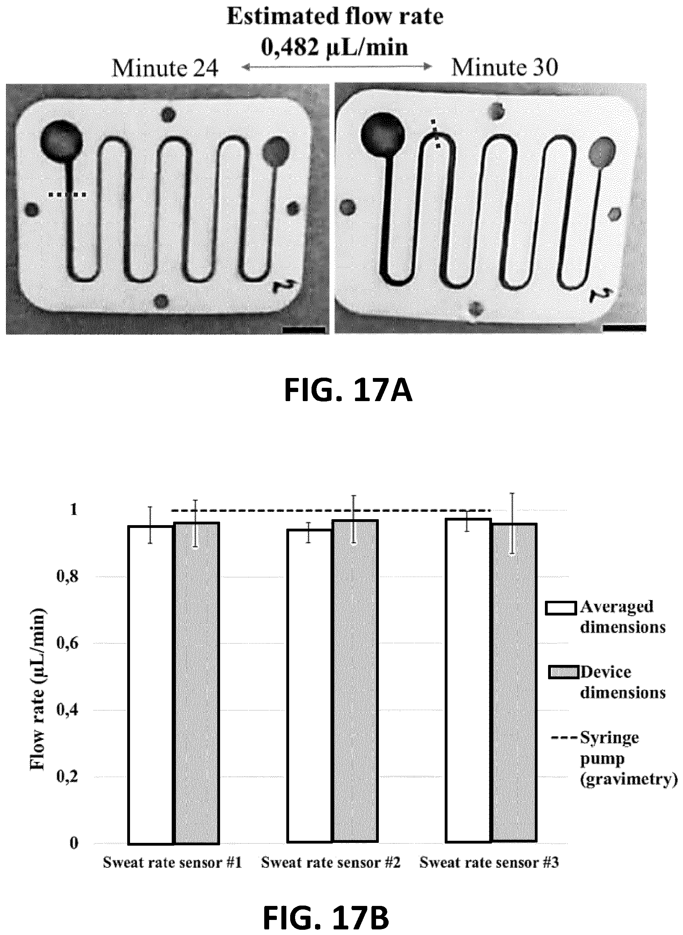

The sweat volume sensor consisted in a microfluidic patch that collected sweat into a microfluidic channel. A dyed paper at the inlet provided colour to the sweat, facilitating the visual determination of the fluid front. Knowing the geometrical dimensions of the microfluidic channel and taking consecutive pictures, the volume of collected sweat for a period of time can be calculated. The variable used in the model, instantaneous sweat rate, was calculated from the increment in volume for a given period of time. In Figure 17A, an in vivo example of the working mechanism of the sensor can be seen. In vitro calibration of this sensor consisted in injecting water using a syringe pump and comparing the flow rate against the set one (Figure 17B). It was also validated that the fabrication method, laser cutting, was replicable enough because there was no significant difference for flow rate calculation when using the averaged dimensions of the microfluidic channel or the individual ones.

### In vivo test

The method for in vivo tests consisted in the following steps:
a) Skin cleaning prior to the attachment of the sampling patches for removing any contaminant and ensure a good adhesion. First, ethanol rinsing using sterile gauzes followed by DI water rinsing to remove completely ethanol which may prevent sweating and a final drying step with sterile gauzes. Attachment of the sampling patches on the chest of the subject as well as the heart rate strap monitor.
b) Starting the test which can be at a cycloergometer or running track. More details on the tests carried out below.
c) Once the subject has started sweating, usually around 10-15 minutes into the test, simultaneous measurements of blood lactate, sweat lactate, sweat rate and heart rate were performed.
d) For blood lactate, a puncture was done at the earlobe to draw blood to perform the measurement. Measurements were done using Lactate Pro2 meter and test strips (Akray, Japan) and also Lactate Plus meter and their test strips (Nova Biomedical, USA). For some subjects, blood was collected in a capillar for later testing with a reference instrumentation at laboratory (colorimetric photometer, Diaglobal, Germany).
e) For sweat lactate, a Lactate Pro2 test strip was placed into the custom reader and the mobile app started to receive the measured signal. The test strip is approached to the capture zone of the sampling patch and when sweat enters to the sensing chamber a peak appears on the measurement screen due to the start of lactate oxidation. Chronoamperometry data can be saved for later analysis with time traceability. The capture zone of the sampling patch was cleaned using cotton swabs of the excess sweat. The test strip was discarded and a new one was used for the next measurement.
f) For the sweat rate measurement, a picture was taken with the smartphone ensuring that the fluid front was distinguishable (it has been noted that flash allows for better contrast). Image was saved for later analysis with time traceability.
g) For the heart rate measurement, the heart rate strap monitor data was transferred to their own mobile app which can be accessed after the test for analysis with time traceability.
h) The process was repeated for each measurement for a given subject until the test was finished with variated frequency depending on the test. Typically, there were from 4 to 8 measurements for subject.

### Field study

Variated physical tests were carried out with the goal of obtaining a wide range of lactate concentrations, subjects for a complete bioequivalence study. Tests were carried out with volunteers, athletes of the Centre d'Alt Rendiment (CAR) and students of the Institut Nacional d'Educació Fisica (INEFC) from February 2021 to April 2021. The dataset contained 152 measurements from 32 subjects. The protocols carried out were intended to mimic the actual tests and trainings carried out by the athletes or volunteers. Apart from the setting (cycloergometer or running track), we could differentiate in terms of intensity between incremental or constant load. It was relevant to mix both types of activity because they produce different responses in lactate levels, increasing the variability of the data and making the posterior analysis more robust.

Subjects:

| | |
|---|---|
| • Age: | 21 ± 4 years |
| • Gender: | 21 men and 11 women |
| • Height: | 1.74 ± 0.09 m |
| • Weight: | 66 ± 9 kg |
| • Training level: | 20 Amateur and 12 Athletes |

Cycloergometer test - Incremental intensity
- 6 subjects, N = 38 measurements, Naverage= 6.33 measurements/subject.
- Warm-up for 10 minutes at 50-100 W power load.
- After warming up, increase of power 30 W for 3 minutes.
- Measurements performed after each period of 3 minutes.
- Increasing up to subject maximum capacity or cycloergometer capacity.
- After maximum reached, 3-minute period of active rest followed by 3-minute period of complete rest. Measurements after each of these periods as well.

Running track test - Constant intensity
- 22 subjects, N = 92 measurements , Naverage= 4.18 measurements/subject.
- Subjects already warmed up.
- Four series of 1200-2000 meters.
- Measurements performed after each series and after resting for 3 minutes. Time between measurements depended on the subject pace but close to 6 minutes.

Triathlon (Cycloergometer-Running Track) test
- 4 subjects, N = 21 measurements, Naverage= 5.25 measurements/subject.
- Subjects already warmed up.
- Cycling for 10 minutes followed by 400 meters running track. Three consecutive series and measurements were performed after each transition and after resting for 3 minutes. Time between measurements depended on the subject pace but close to 10 minutes.

### Data Analysis

The analysis process started by extracting data from the sensors. For the sweat lactate measurements, the averaged current of the stable region of the chronoamperometry was used directly, as its proportionality with lactic acid concentration had been previously demonstrated (Fig. 16). For the sweat rate measurements, the procedure described for in vitro characterization was applied. The heart rate was extracted from the heart rate monitor source for the same test time as the rest of measurements. The lactate Excretion Rate (LER) is the product of the sweat lactate concentration and the sweat rate to account for volume dilution. The LER was divided by the heart rate (HR) to create the ELER (Exertion and Lactate Excretion Rate) parameter as a way to introduce the expected relation between independent variables. The purpose the ELER parameter is to provide the models with an initial logical relation between independent variables. On the contrary, if the relation is not present in the data, model performance would be reduced.

Pre-processing for all models used consisted in centring and scaling for all continuous variable in order to have a mean of 0 and a standard deviation 1. This way, the numerical stability is improved and continuous variables of different values can be used together. The initial set of models used for the prediction of blood lactate are multiparametric linear models including LM (Linear Models), PLS (Partial Least Squares) or PCR (Principal Component Regression). Due to the complexity of the data, it is expected that non-linearity must be included through the use of a neural network algorithm (MLP, MultiLayer Perceptron).

The structure of MLP consists in an input layer (independent variables), a hidden layer and the output layer (dependent variable). MLP just have a single hidden layer with neurons (hidden units) which are mathematical expressions consisting of weighted inputs (obtained from supervised back-propagation training) that produce an output only above a certain threshold. This threshold is controlled by the activation function (which can be of different nature) which is the non-linearity term added to the model. MLP used is from the package caret in R, the number of neurons in the hidden layer was tuned (from 1 to 10, 5 neurons were the final optimized number) and the activation function used was the rectified linear function.

The statistical metrics used to test the accuracy of prediction of the model was RMSE. RMSE (Root Mean Square Error) is the square root of the squared differences between real (blood lactate values) and predicted (from the trained model) values and provides information about the inaccuracy of the prediction in the same units as the variables which facilitates comprehension.

### Blood lactate measurement variability

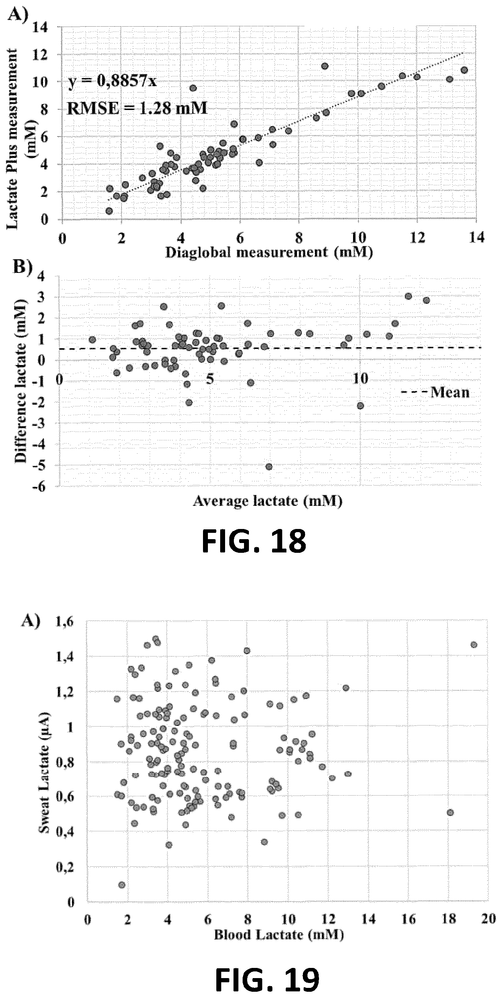

As we measured the same blood sample with the two measurement systems available currently for lactate determination (the colorimetric photometer at laboratory, Diaglobal, and the electrochemical portable meters, Lactate Plus), the variability associated to the field methodology can be studied. There were enough measurements to carry out the cross-correlation, shown in Fig. 18. Fig. 18A Shows a correlation plot between Lactate Plus (portable meter and test strips) and Diaglobal (optical instrument) for blood lactate. Fig. 18B shows a Bland-Altman plot showing the agreement between methodologies.

It was found a significant deviation between the two methodologies of 1.3 mM (RMSE), but this error is accepted by the sports field and it is the standard degree of accuracy required for lactate monitoring tool.

### Bioequivalence

Once the dataset was built, the first step consisted in finding the relationship between blood lactate and sweat lactate. A simple correlation plot was enough to see that direct correlation was not feasible, see Fig. 19, and that multiparametric approaches must be used.

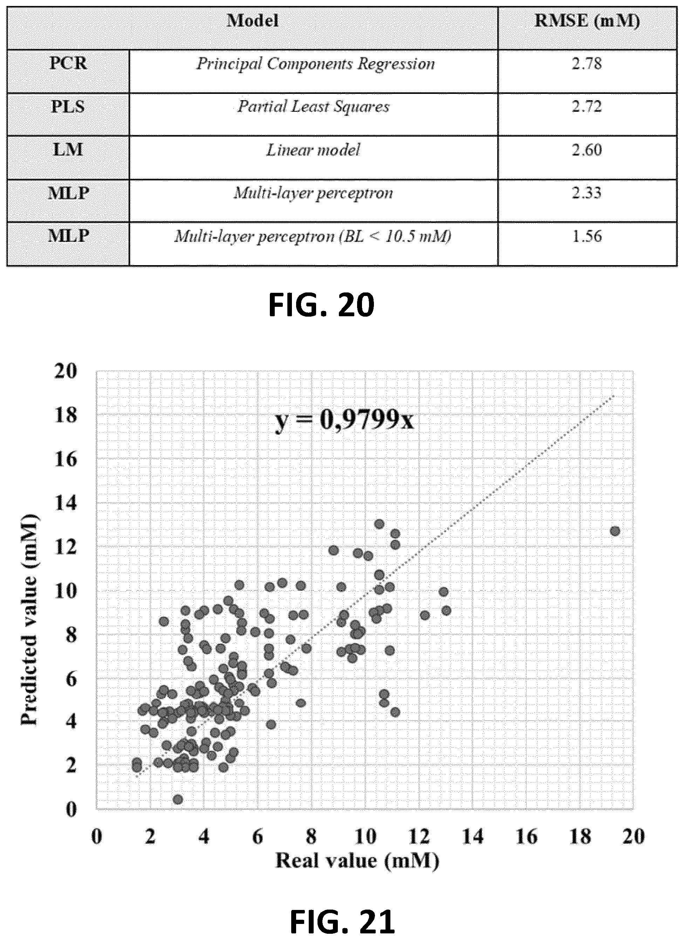

The first set of models applied were multiparametric regression models: PCR, PLS and LM. RSME values can be seen in Fig. 20. All these algorithms are linear, therefore allowing for simpler, less computing intensive predictions. However, the performance obtained initially was not satisfactory enough to use them as a reliable tool for the blood lactate prediction. Not only in the accuracy (reflected by the RMSE value), but the prediction robustness was not reproducible at all, depending greatly on the training data used. On the other hand, MLP showed a robust prediction with minimal variation to training data, as well as an increase in prediction accuracy.

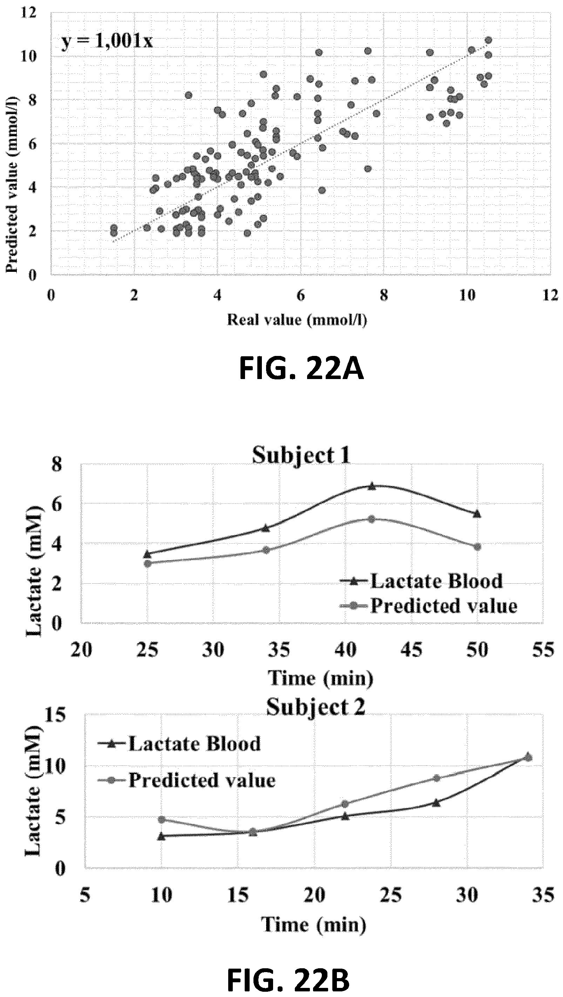

A correlation plot of multiple predictions combined using MLP algorithm is shown in Fig. 21. It can be clearly seen how high blood lactate values are scarce in our dataset and have the least accurate predictions. Besides, the relevance in predicting values of lactate over 10 mM is limited as the athlete would be far past the anaerobic threshold. Therefore, filtering out the high blood lactate values, the prediction performance notably increased as shown in Figure 22A . Not only in absolute terms as it can be seen in the RMSE value (reduced up to 1.56 mM, much closer to the 1.3 mM reference stated previously), but in the capability to follow the temporal evolution of the blood lactate for a given user (Figure 22B).

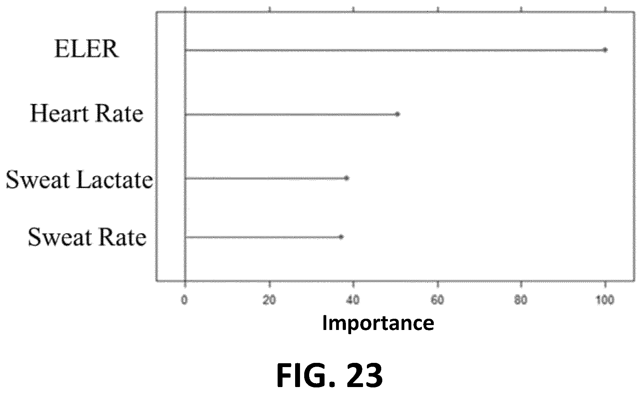

A meaningful characteristic of the MLP model used is the relative importance of each independent variable in the prediction (Fig. 23). The most important parameter was found to be ELER, the derived parameter from the rest of measurements, confirming that the relationship envisioned was correct. The next variables, in terms of importance, were the individual measurements of heart rate, sweat lactate and sweat rate. These results support the idea that the non-invasive measurements carried out are the basis of the lactate bioequivalence results showed in this work, and there are not confounding factors such as subject characteristics or testing practices.

Therefore, the feasibility of predicting blood lactate from non-invasive measurements was validated. The same strategy could be applied to other blood biomarkers, adapted to the relevant parameters to be measured in the context of the application. This type of devices could provide the next leap in sweat monitoring, providing useful and relevant data to both user and professional clinicians.

### CLAUSES

The present application comprises the following clauses:
1.- A wearable device for continuous monitoring of health parameters of a user,
   the device comprising:
   at least one sweat sensor for measuring a sweat biomarker,
   a sweat collection inlet arranged in the device for collecting sweat when the device is worn by a user,
   a microfluidic channel for conveying collected sweat from the inlet to the sweat sensor,
   at least one vital-sign sensor arranged in the device for measuring a vital-sign or physiological sign of the user,
   a sweat volume sensor for measuring volume of the collected sweat, and
   a processing unit adapted for receiving and processing data provided by the sweat sensor, the vital-sign biosensor and the sweat volume sensor;
   wherein the vital-sign sensor comprises at least a heart rate sensor; and
   wherein the processing unit is further adapted to calculate, preferably by means of machine learning algorithms, the concentration of the biomarker in blood based on data provided by: the sweat biomarker sensor, the sweat volume sensor and heart rate sensor.
2.- A wearable device according to clause 1, wherein the sweat biomarker sensor is selected from a list comprising: a sweat lactate sensor and a sweat glucose sensor.
3.- A wearable device according to any of the preceding clauses, wherein the sweat biomarker sensor is a sweat lactate sensor.
4.- A wearable device according to any of the preceding clauses, wherein the sweat biomarker sensor is a sweat lactate sensor, and wherein the processing unit is adapted to calculate, preferably by means of machine learning algorithms, a blood lactate concentration based on data provided by: the sweat lactate sensor, the sweat volume sensor and heart rate sensor.
5.- A wearable device according to any of the preceding clauses, further comprising a communication module adapted for the wireless transmission of data processed by the processing unit.
6.- A wearable device according to any of the preceding clauses further comprising sensing chamber and at least one of the following sensors: a sweat lactate sensor, a sweat conductivity sensor, metabolites sensor, ions sensors, amino acids sensor, and, placed in the sensing chamber, and wherein the microfluidic channel communicates the sweat inlet with the sensing chamber.
7.- A wearable device according to any of the preceding clauses, comprising at least one of the following vital-signs or signs sensors: a heart rate sensor, a respiratory rate sensor, blood pressure sensor, body temperature sensor, and oxygen saturation sensor.
8.- A wearable device according to any of the preceding clauses, wherein the sweat volume sensor comprises a microfluidic circuit or a microfluidic reservoir, fluidly communicated with the sensing chamber and arranged downstream the sensing chamber.
9.- A wearable device according to clause 8, wherein the sweat volume sensor comprises a pair of electrodes and a microfluidic reservoir fluidly communicated with the sensing chamber and arranged relative to each other, such that a capacitance value between the two electrodes is variable depending on the amount of sweat in the reservoir.
10.- A wearable device according to clause 9, wherein the two electrodes are a pair of strips opposite each other, and the microfluidic reservoir is arranged in between the two electrodes, wherein preferably the electrodes are embodied as conductive strips, and more preferably the electrodes are embodied as conductive flexible strips.
11.- A wearable device according to any of the preceding clauses, further comprising:
   a main housing having at least part of the processing unit enclosed therein,
   means for attaching the main housing to a part of the user's body,
   a consumable component configured to be manually attachable and detachable from the main housing, and having a contact surface provided to be in contact with the user's skin when the main housing is attached to a user's body part, and the consumable component is coupled with the main housing,
   wherein the consumable component incorporates: the sweat collection inlet formed in the consumable component's contact surface at least one sweat sensor, the sweat rate measuring device, and the microfluidic channel, and
   electric connection means for electrically connecting the consumable component with the processing unit when the consumable component is coupled with the main housing.
12.- A wearable device according to clause 11, wherein the means for attaching the main housing to a part of a user's body, comprises a flexible band having two ends respectively couplable with the main housing, and wherein the flexible band is fitted with at least one biosensor for measuring a vital-sign or physiological sign of the user.
13.- A wearable device according to clause 11, wherein the means for attaching the main housing to a part of a user's body, comprises an adhesive surface suitable to be adhered on a user's skin, and wherein preferably the adhesive surface is provided on the consumable component's contact surface.
14.- A wearable device according to any of the preceding clauses, further comprising a sweat rate sensor, and wherein the processing unit is further adapted for monitoring user fatigue.
15.- A wearable device according to any of the preceding clauses, further comprising a conductivity sensor and a ion sensor, and wherein the processing unit is further adapted for monitoring dehydration in athletes, based on data provided by the sweat volume sensor, the conductivity sensor and the ion sensor.
16.- A wearable device according to any of the preceding clauses, wherein the sweat biomarker sensor is a sweat glucose sensor and further comprising: a conductivity sensor, and wherein the processing unit is adapted to calculate, preferably by means of machine learning algorithms, a blood glucose concentration based on data provided by the heart rate sensor, the sweat volume sensor, the conductivity sensor and the sweat glucose sensor.
17.- A wearable device according to clause 16, wherein the processing unit is further adapted to monitor the nocturnal hypoglycaemia, based on data provided by the heart rate sensor, the sweat volume sensor, the conductivity sensor and the sweat glucose sensor preferably wherein the sweat glucose sensor is adapted to detect glucose sweat concentrations below 55mg/L.
18.- A wearable device according to any of the preceding clauses, wherein the sweat biomarker sensor is a sweat lactate sensor and/or a sweat glucose sensor, the device further comprising an accelerometer, a conductivity sensor, and an ions sensor preferably a sodium sensor, and wherein the processing unit is further adapted to monitor the peritoneal dialysis based on data provided by the heart rate sensor, the accelerometer, the sweat rate sensor, the conductivity sensor, the ions sensor and the sweat lactate sensor and/or the sweat glucose sensor.
19.- A method for non-invasively determining the concentration of a biomarker in blood, the method comprising:
   a) receiving the concentration of such biomarker in sweat, the volume of sweat received and the heart rate of the subject,
   b) computing through a multiparametric regression model a value representing the concentration of the biomarker in blood, and
   c) determining the computed value is the concentration of the biomarker in blood.
20.- The method according to clause 19, wherein the biomarker is selected from a list comprising: lactate and glucose.
21.- The method according to clauses 19 or 20, wherein the biomarker is lactate.
22.- The method according to any of the clauses 19 to 21, wherein the biomarker is lactate and the method comprises determining the blood lactate concentration based on the sweat lactate, the sweat volume and the heart rate.
23.- The method according to any of the clauses 19 to 22, wherein the concentration of the biomarker in sweat, the volume of sweat received, and/or the heart rate of the subject are received from a wearable device according to any of the clauses 1 to 18.
24.- A method for non-invasively evaluating the condition of a subject, the method comprising determining the concentration of a biomarker in blood in a non-invasive or minimally invasive way, based on the concentration of such biomarker in sweat.
25.- The method according to clause 24, wherein the method comprises determining the concentration of the biomarker in blood based on the concentration of such biomarker in sweat, the sweat volume, and the heart rate of the subject.
26.- The method according to clauses 24 or 25, wherein the biomarker is selected from a list comprising lactate and glucose.
27.- The method according to clauses 24 to 26, wherein the biomarker is lactate.
28.- The method according to any of the clauses 24 to 27, wherein the concentration of the biomarker in sweat, the volume of sweat received, and/or the heart rate of the subject are received from a wearable device according to any of the clauses 1 to 18.

### More particularly, the present application comprises the following clauses:

1.- A wearable device (1) for continuous monitoring of health parameters of a user,
   the device (1) comprising:
   at least one sweat sensor for measuring a sweat biomarker,
   a sweat collection inlet (11) arranged in the device (1) for collecting sweat when the device (1) is worn by a user,
   a microfluidic channel (22) for conveying collected sweat from the inlet to the sweat sensor,
   at least one vital-sign sensor arranged in the device (1) for measuring a vital-sign or physiological sign of the user,
   a sweat volume sensor (18) for measuring volume of the collected sweat, and
   a processing unit (7) adapted for receiving and processing data provided by the sweat biomarker sensor, the vital-sign biosensor and the sweat volume sensor (18);
   wherein the vital-sign sensor comprises at least a heart rate sensor;
      and
   wherein the sweat biomarker sensor is a sweat lactate sensor and wherein the processing unit (7) is further adapted to calculate, preferably by means of machine learning algorithms, a blood lactate concentration based on data provided by: the sweat lactate sensor, the sweat volume sensor(18) and heart rate sensor.
2.- A wearable device (1) according to clause 1 , further comprising a communication module adapted for the wireless transmission of data processed by the processing unit (7).
3.- A wearable device (1) according to any of the preceding clauses further comprising a sensing chamber (21), wherein the sweat lactate sensor is placed in the sensing chamber (21), and wherein the microfluidic channel (22) communicates the sweat inlet with the sensing chamber (21).
4.- A wearable device (1) according to clause 3, further comprising at least one of the following sensors: a sweat conductivity sensor, metabolites sensor, ions sensor, and amino acids sensor, placed in the sensing chamber (21).
5.- A wearable device (1) according to any of the preceding clauses, comprising at least one of the following further vital-signs or signs sensors: a respiratory rate sensor, blood pressure sensor, body temperature sensor, and oxygen saturation sensor.
6.- A wearable device (1) according to any of the preceding clauses, wherein the sweat volume sensor (18) comprises a microfluidic circuit or a microfluidic reservoir (19), fluidly communicated with the sensing chamber (21) and arranged downstream the sensing chamber (21).
7.- A wearable device (1) according to clause 6, wherein the sweat volume sensor (18) comprises a pair of electrodes (20,20') and a microfluidic reservoir (19) fluidly communicated with the sensing chamber (21) and arranged relative to each other, such that a capacitance value between the two electrodes (20,20') is variable depending on the amount of sweat in the reservoir, preferably wherein the two electrodes (20,20') are a pair of strips opposite each other, and the microfluidic reservoir (19) is arranged in between the two electrodes (20,20'), wherein more preferably the electrodes (20,20') are embodied as conductive strips, and more preferably the electrodes (20,20') are embodied as conductive flexible strips.
8.- A wearable device (1) according to any of the preceding clauses, further comprising:
   a main housing (2) having at least part of the processing unit (7) enclosed therein,
   means (3) for attaching the main housing (2) to a part of the user's body,
   a consumable component (5) configured to be manually attachable and detachable from the main housing (2), and having a contact surface (6) provided to be in contact with the user's skin when the main housing (2) is attached to a user's body part, and the consumable component is coupled with the main housing (2),
   wherein the consumable component (5) incorporates: the sweat collection inlet (11) formed in the consumable component's contact surface (6) at least one sweat sensor, a sweat rate measuring device, and the microfluidic channel (22), and
   electric connection means (12) for electrically connecting the consumable component (5) with the processing unit (7) when the consumable component (5) is coupled with the main housing (2).
9.- A wearable device (1) according to clause 8, wherein the means (3) for attaching the main housing (2) to a part of a user's body, comprises a flexible band having two ends respectively couplable with the main housing (2), and wherein the flexible band is fitted with at least one biosensor for measuring a vital-sign or physiological sign of the user.
10.- A wearable device (1) according to clause 8, wherein the means (3) for attaching the main housing (2) to a part of a user's body, comprises an adhesive surface suitable to be adhered on a user's skin, and wherein preferably the adhesive surface is provided on the consumable component's contact surface (6).
11.- A wearable device (1) according to any of the preceding clauses, further comprising a sweat rate sensor, and wherein the processing unit (7) is further adapted for monitoring user fatigue.
12.- A wearable device (1) according to any of the preceding clauses, further comprising a conductivity sensor and an ion sensor, and wherein the processing unit (7) is further adapted for monitoring dehydration in athletes, based on data provided by the sweat volume sensor (18), the conductivity sensor and the ion sensor.
13.- A wearable device (1) according to any of the preceding clauses, wherein the sweat biomarker sensor is a sweat glucose sensor and further comprising: a conductivity sensor, and wherein the processing unit (7) is adapted to calculate, preferably by means of machine learning algorithms, a blood glucose concentration based on data provided by the heart rate sensor, the sweat volume sensor (18), the conductivity sensor and the sweat glucose sensor; preferably wherein the processing unit (7) is further adapted to monitor the nocturnal hypoglycaemia, based on data provided by the heart rate sensor, the sweat volume sensor (18), the conductivity sensor and the sweat glucose sensor preferably wherein the sweat glucose sensor is adapted to detect glucose sweat concentrations below 55mg/L.
14.- A wearable device (1) according to any of the preceding clauses, wherein the sweat biomarker sensor is a sweat lactate sensor and/or a sweat glucose sensor, the device (1) further comprising an accelerometer, a conductivity sensor, and an ions sensor preferably a sodium sensor, and wherein the processing unit (7) is further adapted to monitor the peritoneal dialysis based on data provided by the heart rate sensor, the accelerometer, the sweat rate sensor, the conductivity sensor, the ions sensor and the sweat lactate sensor and/or the sweat glucose sensor.
15.- A computer-implemented method for non-invasively determining the concentration of a biomarker in blood in a wearable device (1) according to any one of clauses 1 to 14, the method comprising:
   d) receiving the concentration of such biomarker in sweat, the volume of sweat collected and the heart rate of the subject,
   e) computing through a multiparametric regression model a value representing the concentration of the biomarker in blood, and
   f) determining the computed value is the concentration of the biomarker in blood
   wherein the biomarker is lactate and the method comprises determining the blood lactate concentration based on the sweat lactate, the sweat volume and the heart rate.

## Claims

1. A wearable device (1) for continuous monitoring of health parameters of a user,
the device (1) comprising:
at least one sweat sensor for measuring a sweat biomarker,
a sweat collection inlet (11) arranged in the device (1) for collecting sweat when the device (1) is worn by a user,
a microfluidic channel (22) for conveying collected sweat from the inlet to the sweat sensor,
at least one vital-sign sensor arranged in the device (1) for measuring a vital-sign or physiological sign of the user,
a sweat volume sensor (18) for measuring volume of the collected sweat, and
a processing unit (7) adapted for receiving and processing data provided by the sweat sensor, the vital-sign biosensor and the sweat volume sensor (18);
wherein the vital-sign sensor comprises at least a heart rate sensor; and
wherein the processing unit (7) is further adapted to calculate, preferably by means of machine learning algorithms, the concentration of the biomarker in blood based on data provided by: the sweat biomarker sensor, the sweat volume sensor (18) and heart rate sensor.

2. A wearable device (1) according to claim 1, wherein the sweat biomarker sensor is selected from a list comprising: a sweat lactate sensor and a sweat glucose sensor.

3. A wearable device (1) according to any of the preceding claims, wherein the sweat biomarker sensor is a sweat glucose sensor.

4. A wearable device (1) according to any of the preceding claims, wherein the sweat biomarker sensor is a sweat lactate sensor.

5. A wearable device (1) according to any of the preceding claims , further comprising a communication module adapted for the wireless transmission of data processed by the processing unit (7).

6. A wearable device (1) according to any of the preceding claims further comprising sensing chamber (21) and at least one of the following sensors: a sweat lactate sensor, a sweat conductivity sensor, metabolites sensor, ions sensors, amino acids sensor, and, placed in the sensing chamber (21), and wherein the microfluidic channel (22) communicates the sweat inlet with the sensing chamber (21).

7. A wearable device (1) according to any of the preceding claims, comprising at least one of the following vital-signs or signs sensors: a heart rate sensor, a respiratory rate sensor, blood pressure sensor, body temperature sensor, and oxygen saturation sensor.

8. A wearable device (1) according to any of the preceding claims, wherein the sweat volume sensor (18) comprises a microfluidic circuit or a microfluidic reservoir (19), fluidly communicated with the sensing chamber (21) and arranged downstream the sensing chamber (21), preferably wherein the sweat volume sensor (18) comprises a pair of electrodes (20, 20') and a microfluidic reservoir (19) fluidly communicated with the sensing chamber (21) and arranged relative to each other, such that a capacitance value between the two electrodes (20, 20') is variable depending on the amount of sweat in the reservoir, more preferably wherein the two electrodes (20, 20') are a pair of strips opposite each other, and the microfluidic reservoir (19) is arranged in between the two electrodes (20, 20'), wherein preferably the electrodes (20, 20') are embodied as conductive strips, and more preferably the electrodes (20, 20') are embodied as conductive flexible strips.

9. A wearable device (1) according to any of the preceding claims, further comprising:
a main housing (2) having at least part of the processing unit (7) enclosed therein,
means (3) for attaching the main housing (2) to a part of the user's body,
a consumable component (5) configured to be manually attachable and detachable from the main housing (2), and having a contact surface (6) provided to be in contact with the user's skin when the main housing (2) is attached to a user's body part, and the consumable component (5) is coupled with the main housing (2),
wherein the consumable component (5) incorporates: the sweat collection inlet formed in the consumable component's contact surface (6) at least one sweat sensor, the sweat rate measuring device, and the microfluidic channel (22), and
electric connection means (12) for electrically connecting the consumable component (5) with the processing unit (7) when the consumable component (5) is coupled with the main housing (2).

10. A wearable device (1) according to any of the preceding claims, further comprising a sweat rate sensor, and wherein the processing unit (7) is further adapted for monitoring user fatigue.

11. A wearable device (1) according to any of the preceding claims, further comprising a conductivity sensor and an ion sensor, and wherein the processing unit (7) is further adapted for monitoring dehydration in athletes, based on data provided by the sweat volume sensor (18), the conductivity sensor and the ion sensor.

12. A wearable device (1) according to any of the preceding claims, wherein the sweat biomarker sensor is a sweat glucose sensor and further comprising: a conductivity sensor, and wherein the processing unit (7) is adapted to calculate, preferably by means of machine learning algorithms, a blood glucose concentration based on data provided by the heart rate sensor, the sweat volume sensor (18), the conductivity sensor and the sweat glucose sensor, preferably wherein the processing unit (7) is further adapted to monitor the nocturnal hypoglycaemia, based on data provided by the heart rate sensor, the sweat volume sensor (18), the conductivity sensor and the sweat glucose sensor preferably wherein the sweat glucose sensor is adapted to detect glucose sweat concentrations below 55mg/L.

13. A wearable device (1) according to any of the preceding claim, wherein the sweat biomarker sensor is a sweat lactate sensor and/or a sweat glucose sensor, the device further comprising an accelerometer, a conductivity sensor, and an ions sensor preferably a sodium sensor, and wherein the processing unit (7) is further adapted to monitor the peritoneal dialysis based on data provided by the heart rate sensor, the accelerometer, the sweat rate sensor, the conductivity sensor, the ions sensor and the sweat lactate sensor and/or the sweat glucose sensor.

14. A computer-implemented method for non-invasively determining the concentration of a biomarker in blood, the method comprising:
g) receiving the concentration of such biomarker in sweat, the volume of sweat received and the heart rate of the subject,
h) computing through a multiparametric regression model a value representing the concentration of the biomarker in blood, and
i) determining the computed value is the concentration of the biomarker in blood.

15. The method according to claim 14, wherein the biomarker is selected from a list comprising: lactate and glucose.
